# EUROPEAN PATENT APPLICATION

(11) **EP 3 216 857 A1**
(43) Date of publication of application: **13.09.2017**
(21) Application number: 15857361.8
(22) Date of filing: 05.11.2015
(51) Int. Cl.: C12M 3/00, C12M 1/00, C12M 1/08

(54) **CELL CULTURE SYSTEM AND CELL CULTURE CONTAINER**

(30) Priority: 06.11.2014 JP 2014225781
(71) Applicant: OLYMPUS CORPORATION, Hachioji-shi Tokyo 192-8507 (JP)
(72) Inventor: KIMURA, Hiroyuki, Tokyo 192-8507 (JP); MINAMI, Tatsuya, Tokyo 192-8507 (JP); MAKARA, Yasunori, Tokyo 192-8507 (JP)
(74) Representative: Schicker, Silvia
(86) International application number: PCT/JP2015/081200
(87) International publication number: WO 2016/072460

(57) **Abstract**

For the purpose of easily and automatically replacing a culture medium in a cell culture vessel installed in an incubator, a cell culture system of the present invention includes: a culture-medium storage means (3) that holds a culture medium for culturing cells; a temporary holding means (4) that is coupled to the culture-medium storage means (3) and that is provided with a holding space for temporarily holding the culture medium supplied from the culture-medium storage means (3) and a discharge port for discharging the culture medium; a culture-medium supply means (6) that is coupled to the discharge port of the temporary holding means (4) and that supplies the culture medium discharged from the temporary holding means (4), to a cell culture vessel (7); and a discharge means (8) that discharges the culture medium from the cell culture vessel (4), wherein the culture-medium supply means is provided with a culture-medium intermittent supply mechanism that intermittently supplies the culture medium discharged from the temporary holding means, to the cell culture vessel (4) in a predetermined cycle.

## Description

### {Technical Field}

The present invention relates to a cell culture system and a cell culture vessel capable of automatically replacing a culture medium in the cell culture vessel.

### {Background Art}

In recent years, with the progress of stem-cell research and regenerative medicine, it is required to prepare a large amount of cells for clinical use. To prepare cells for clinical use, it is required to work in an environment that conforms to strict standards, and thus, when a worker walks into a work space, there arise the trouble of having to change into disposable work clothes and the costs thereof. Furthermore, a working process performed by the worker becomes a chance of the cultivation system becoming contaminated. Therefore, it is required to reduce, as much as possible, the number of times the worker walks into the work space and does work and to perform possible work automatically instead of manually.

Although periodic replacement of a culture medium (cell culture solution) is required to culture cells, culture-medium replacement carries a risk of contamination of the cultivation system, and thus, it is preferable, as far as possible, to automatically perform culture-medium replacement, without human intervention. As systems for automatically replacing a culture medium, a system in which a transfer robot moves a culture vessel between an incubator and a culture-medium replacement robot is known (PTL 1).

### {Citation List}

### {Patent Literature}

{PTL 1} Japanese Unexamined Patent Application, Publication No. 2002-262856

### {Summary of Invention}

### {Technical Problem}

Because strict standards are set to prepare cells for clinical use, and the risk of contamination of the cultivation system needs to be eliminated as much as possible, automation of the process of culture-medium replacement leads to an advantage; however, in the automatic culture-medium replacement system of PTL 1, because the transfer robot etc. is adopted, the system becomes extremely complicated, thus increasing the risk of developing a system error. Furthermore, because the culture vessel is taken in and out from the incubator, cells in the culture vessel are stressed due to temperature changes. When the system is installed in the incubator in order to avoid this, because the inside of the incubator is a high-humidity environment, there is a risk of trouble developing in mechanical and electrical components, and thus, it is preferable that the system components in the incubator have a simple configuration.

The present invention has been made in view of the above-described circumstances, and provided are a cell culture system and a cell culture vessel capable of easily and automatically replacing a culture medium in a cell culture vessel in the cell culture space and of reducing the risk of developing system trouble by making the system have a simple configuration.

### {Solution to Problem}

According to one aspect, the present invention provides a cell culture system including: a culture-medium storage means that holds a culture medium for culturing cells; a temporary holding means that is coupled to the culture-medium storage means and that is provided with a holding space for temporarily holding the culture medium supplied from the culture-medium storage means and a discharge port for discharging the culture medium; a culture-medium supply means that is coupled to the discharge port of the temporary holding means and that supplies the culture medium discharged from the temporary holding means, to a cell culture vessel; and a discharge means that discharges the culture medium from the cell culture vessel, wherein the cell culture vessel is coupled to the culture-medium supply means at a position lower in the direction of gravitational force than the culture-medium supply means; and the culture-medium supply means is provided with a flow passage that allows the culture medium to be intermittently supplied from the temporary holding means to the cell culture vessel according to the siphon principle.

According to this aspect, even when the user is not present, a solution, such as a culture medium, can be automatically supplied to and discharged from the cell culture vessel. The siphon principle is used, thus allowing the solution, such as a culture medium, to be intermittently supplied to the cell culture vessel, with a simple configuration.

According to another aspect, the present invention provides a cell culture system including: a culture-medium storage means that holds a culture medium for culturing cells; a temporary holding means that is coupled to the culture-medium storage means and that is provided with a holding space for temporarily holding the culture medium supplied from the culture-medium storage means and a discharge port for discharging the culture medium; a culture-medium supply means that is coupled to the discharge port of the temporary holding means and that supplies the culture medium discharged from the temporary holding means, to a cell culture vessel; and a discharge means that discharges the culture medium from the cell culture vessel, wherein the cell culture vessel is coupled to the culture-medium supply means at a position lower in the direction of gravitational force than the culture-medium supply means; and the culture-medium supply means is provided with a supply gate that opens a flow passage when the culture medium in the holding space reaches a predetermined amount.

According to this aspect, even when the user is not present, a solution, such as a culture medium, can be automatically supplied to and discharged from the cell culture vessel. The gate, which opens and closes according to the amount of the culture medium in the temporary holding means, is adopted, thus allowing the solution, such as a culture medium, to be intermittently supplied to the cell culture vessel, with a simple configuration.

Furthermore, in the above-described aspect, the culture-medium supply means may be provided with the flow passage that causes the culture medium before being supplied to the cell culture vessel to pass through a position that is higher in the direction of gravitational force than the discharge port of the temporary holding means and that is lower in the direction of gravitational force than the maximum height of the holding space.

Furthermore, in the above-described aspect, the discharge port may be disposed in a side surface of the temporary holding means at the position of a predetermined height from a bottom surface of the temporary holding means; and the culture-medium supply means may form the flow passage from the discharge port to the outside of the temporary holding means.

Furthermore, in the above-described aspect, the culture-medium supply means may form the flow passage from the discharge port of the temporary holding means to the inside of the temporary holding means; and an end of the culture-medium supply means that is opposite from an end thereof that is connected to the discharge port of the temporary holding means may have an opening in the vicinity of the bottom surface of the temporary holding means with a gap therebetween.

Furthermore, in the above-described aspect, the supply gate may open the flow passage when the culture medium in the holding space reaches a predetermined weight or when the top surface of the culture medium in the holding space reaches a predetermined height. Accordingly, with a simple configuration, the solution, such as a culture medium, can be intermittently supplied to the cell culture vessel.

Furthermore, in the above-described aspect, the cell culture vessel may be provided with a discharge port for discharging the culture medium to the outside when the height of the top surface of the culture medium therein reaches a predetermined height. Accordingly, with a simple configuration, the solution, such as a culture medium, can be automatically supply to and discharged from the cell culture vessel.

Furthermore, in the above-described aspect, it is possible to further include a supply-rate adjusting means that controls the supply rate of the culture medium supplied from the culture-medium storage means to the temporary holding means. Accordingly, the cycle of intermittent supply of the culture medium to the cell culture vessel can be arbitrarily set.

According to still another aspect, the present invention provides a cell culture vessel including: a supply port for supplying a culture medium; a discharge port for discharging the culture medium; and a tubular member that is connected to the discharge port, wherein the tubular member forms a flow passage that allows the culture medium in the cell culture vessel to be intermittently discharged according to the siphon principle.

According to this aspect, the tubular member may form a flow passage that passes through a position that is higher in the direction of gravitational force than the discharge port and that is lower in the direction of gravitational force than the maximum height of a space in the cell culture vessel.

Furthermore, in the above-described aspect, the discharge port may be disposed in a side surface of the cell culture vessel at the position of a predetermined height from the bottom surface of the cell culture vessel; and the tubular member may form a flow passage from the discharge port to the outside of the cell culture vessel.

Furthermore, in the above-described aspect, the tubular member may form a flow passage from the discharge port to the inside of the cell culture vessel; and an end of the tubular member that is opposite from an end thereof that is connected to the discharge port may have an opening in the vicinity of the bottom surface of the cell culture vessel with a gap therebetween.

According to still another aspect, the present invention provides a cell culture system including: the above-described cell culture vessel; and a culture-medium supply means that supplies the culture medium from the supply port of the cell culture vessel to the inside thereof.

### {Advantageous Effects of Invention}

According to the present invention, it is possible to replace a culture medium even when a worker is not present and to reduce the number of times the worker walks into a work space. Accordingly, it is possible to save the trouble of having to change into disposable work clothes and the costs thereof and to reduce the risk of contamination of a cell culture system by bacteria etc. Furthermore, because the configuration of the system is simple, it is possible to reduce the risk of a system error and to avoid the risk of an impact on culture conditions due to an error.

### {Brief Description of Drawings}

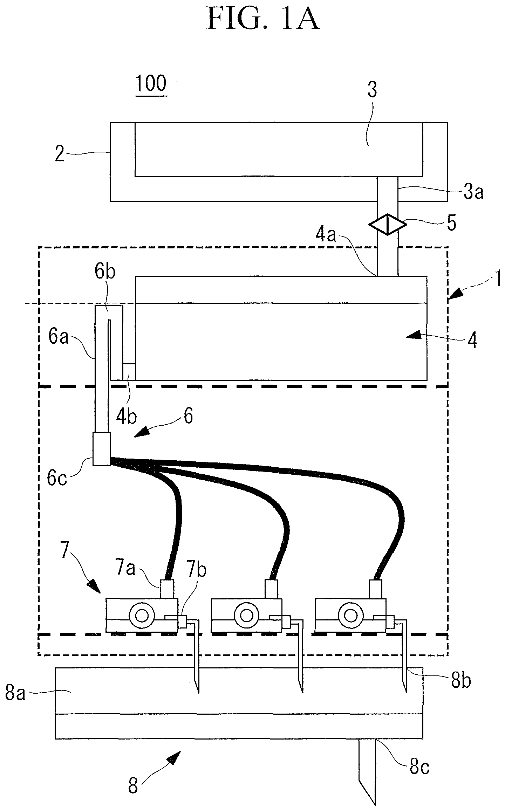

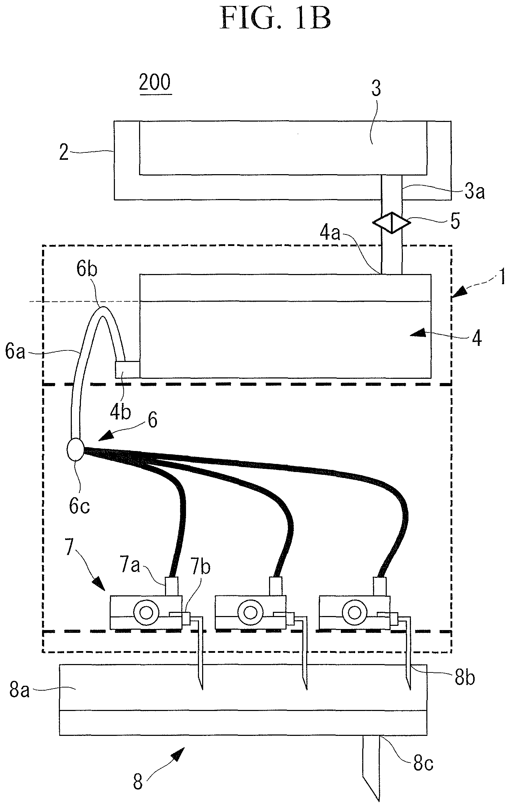

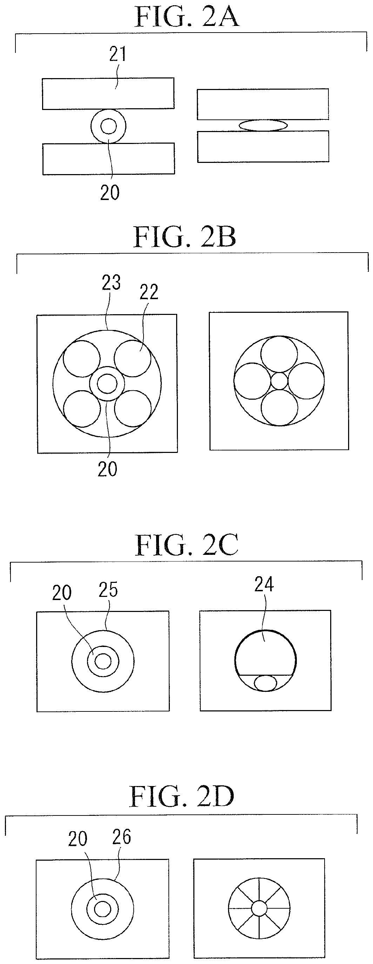

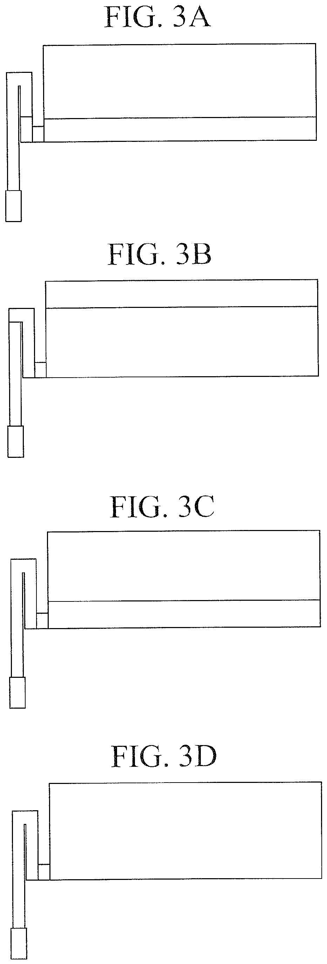

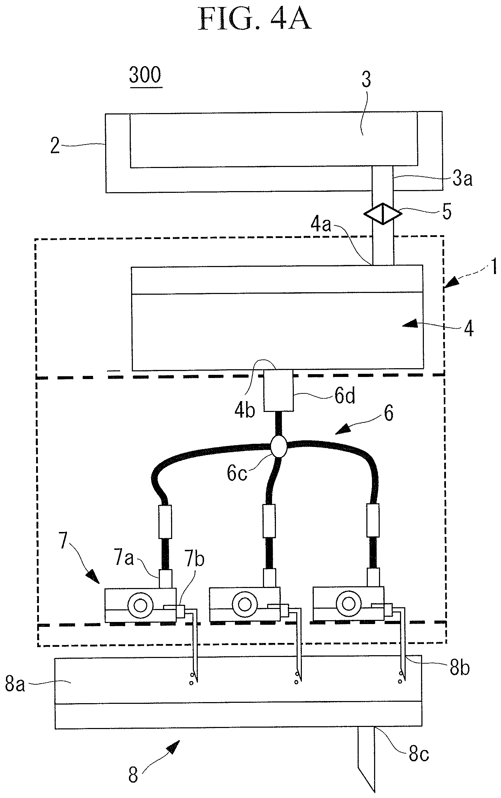

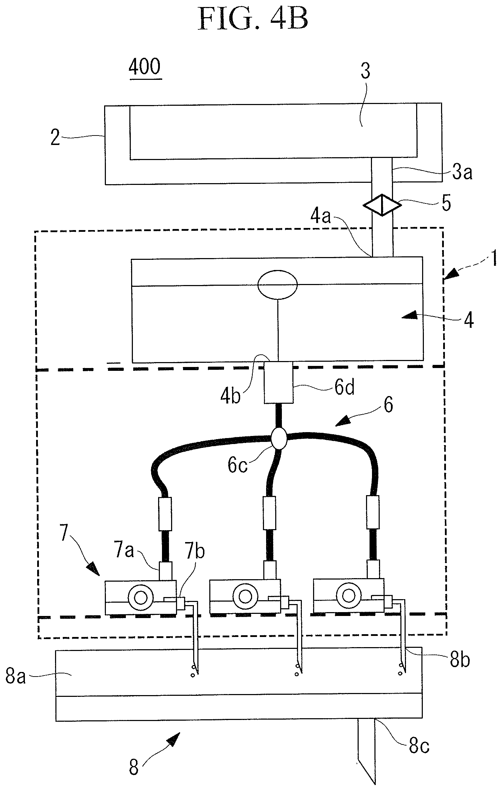

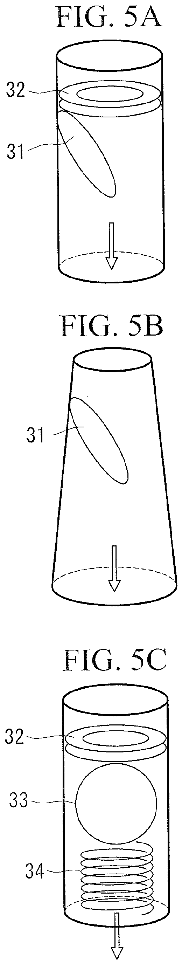

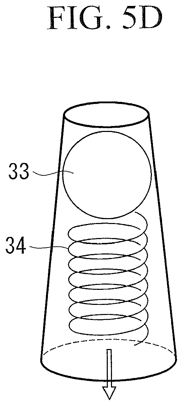

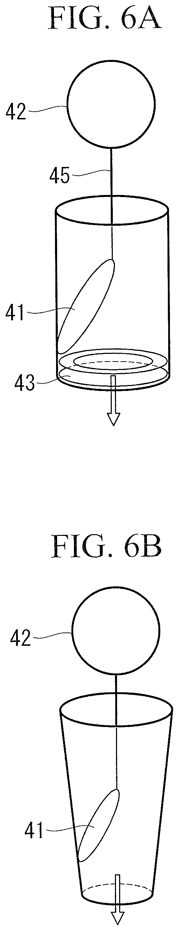

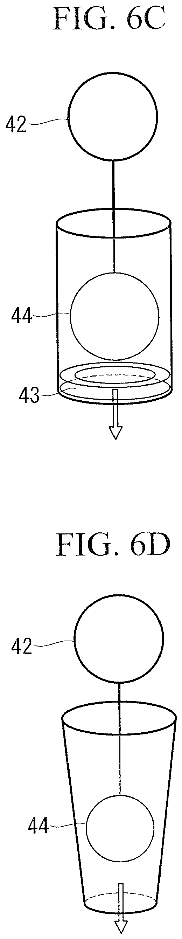

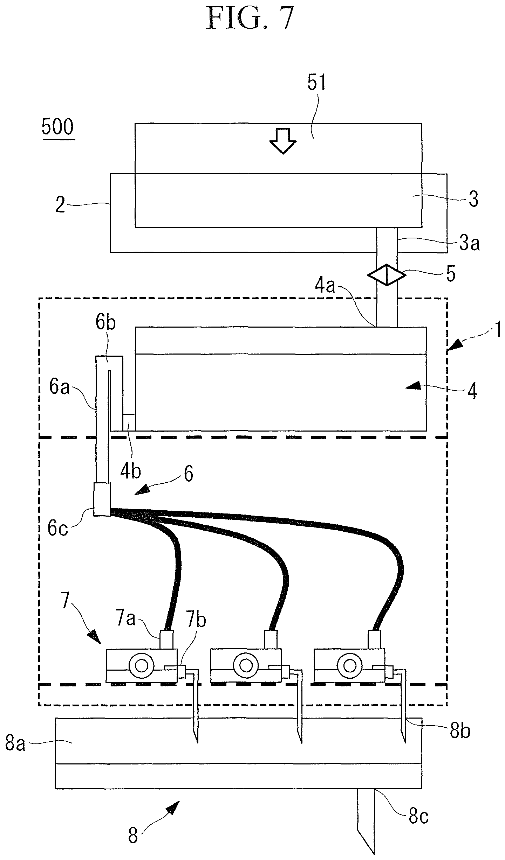

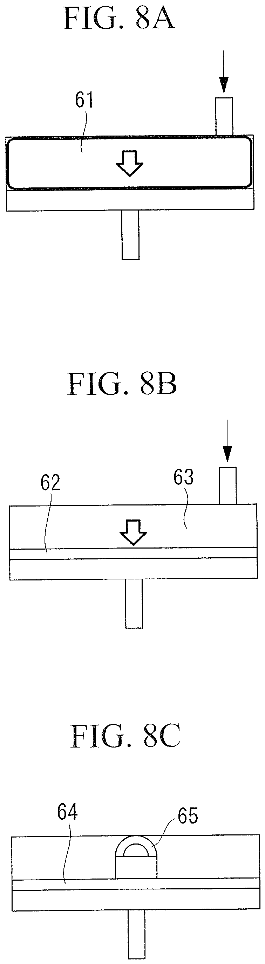

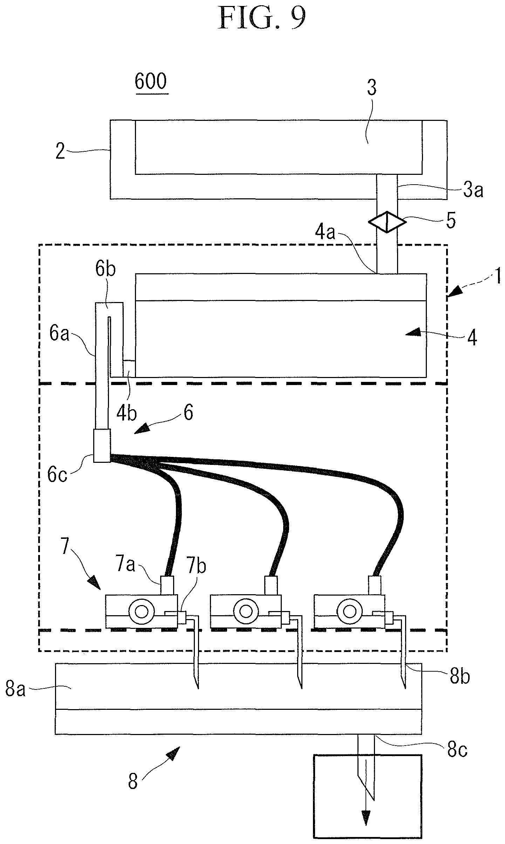

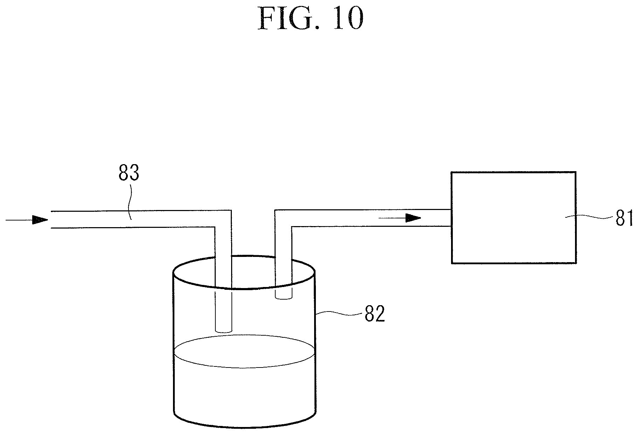

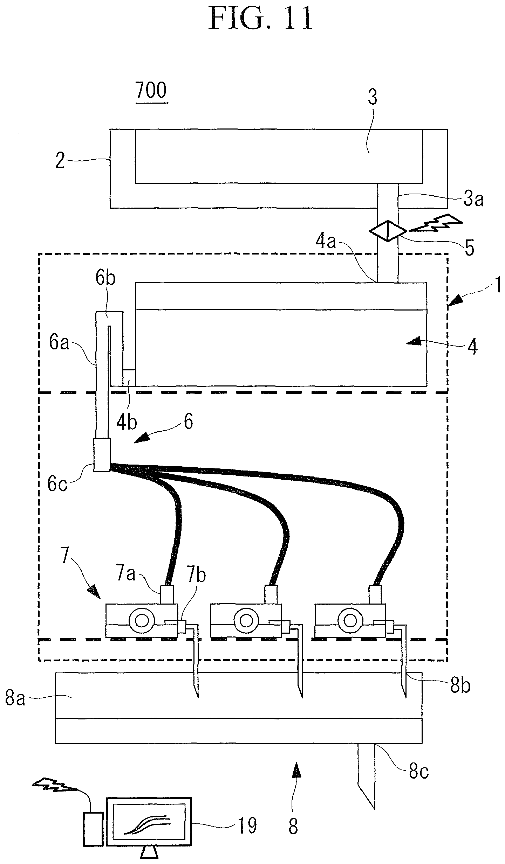

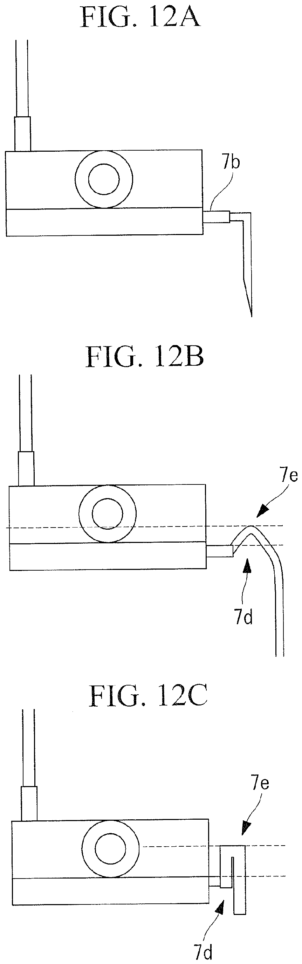

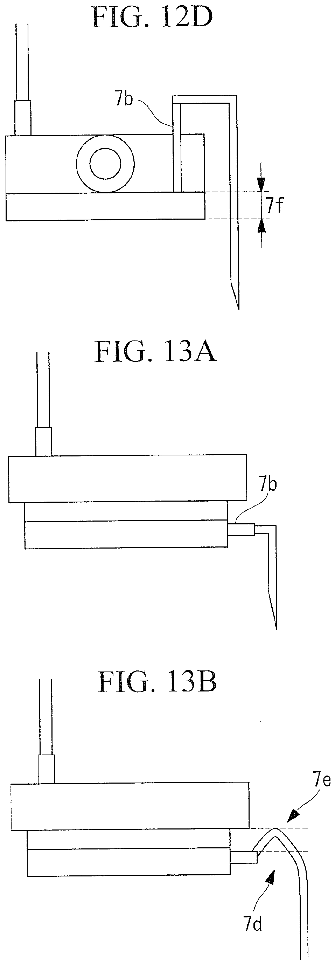

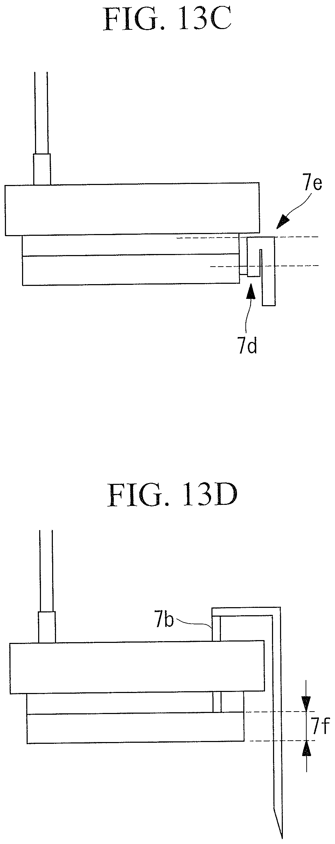

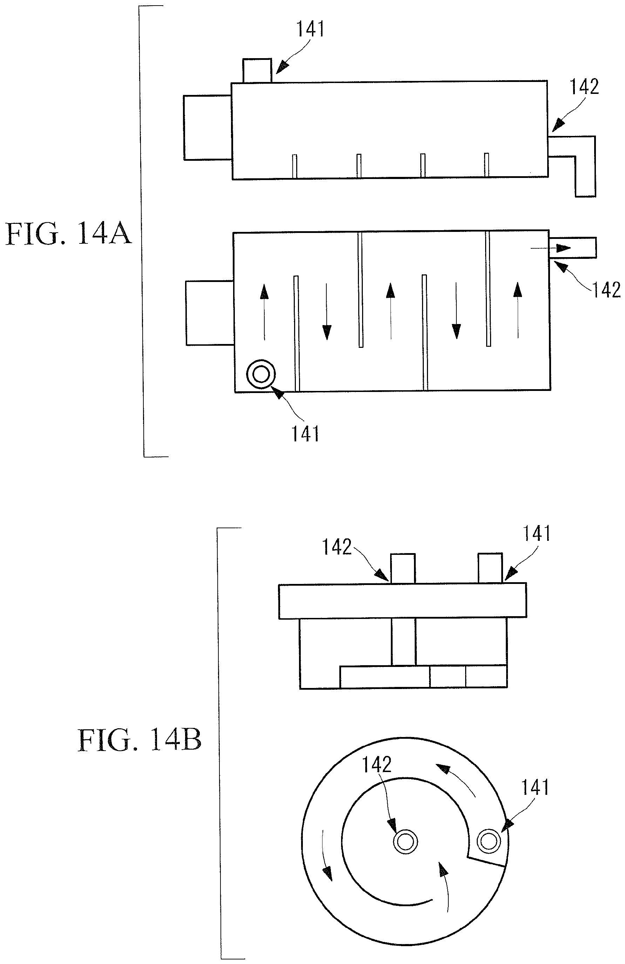

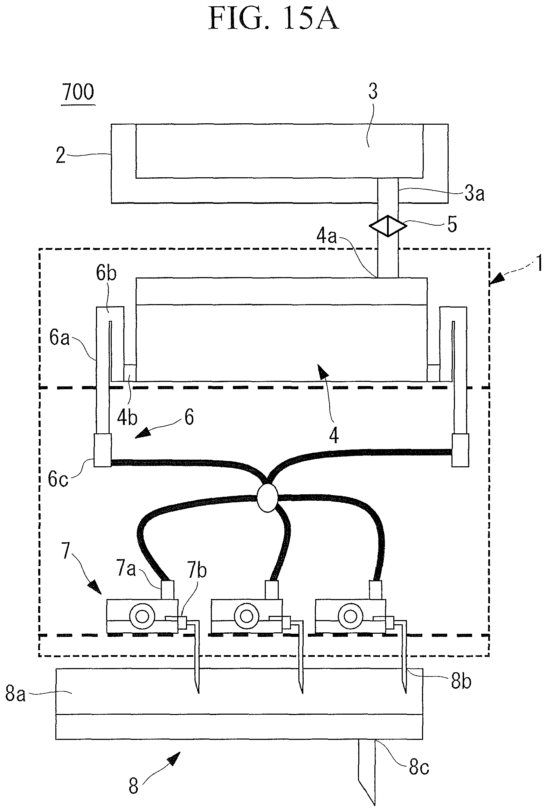

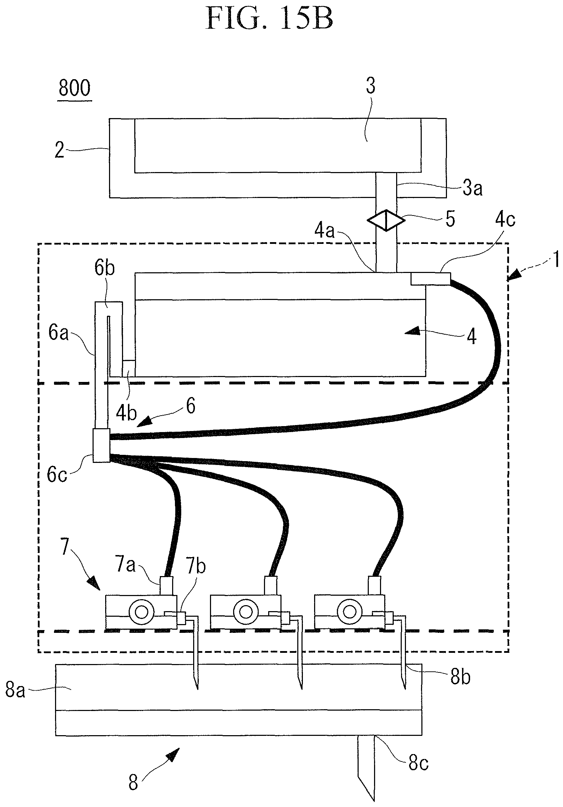

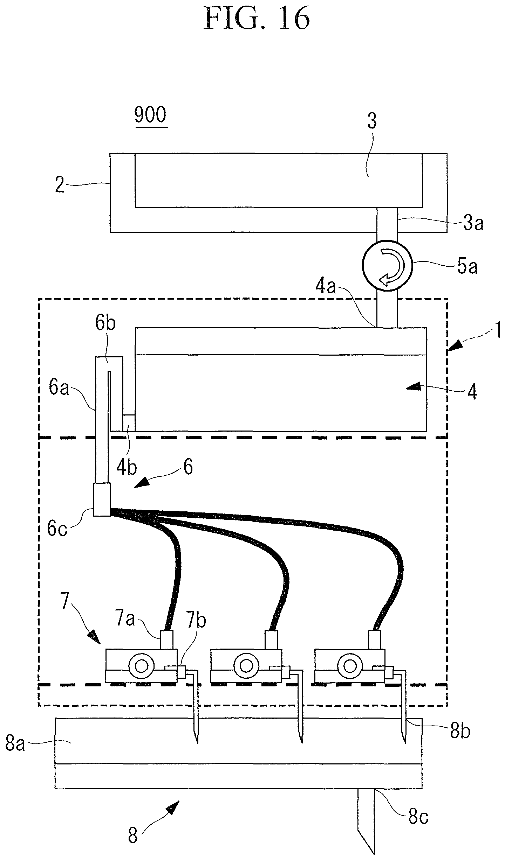

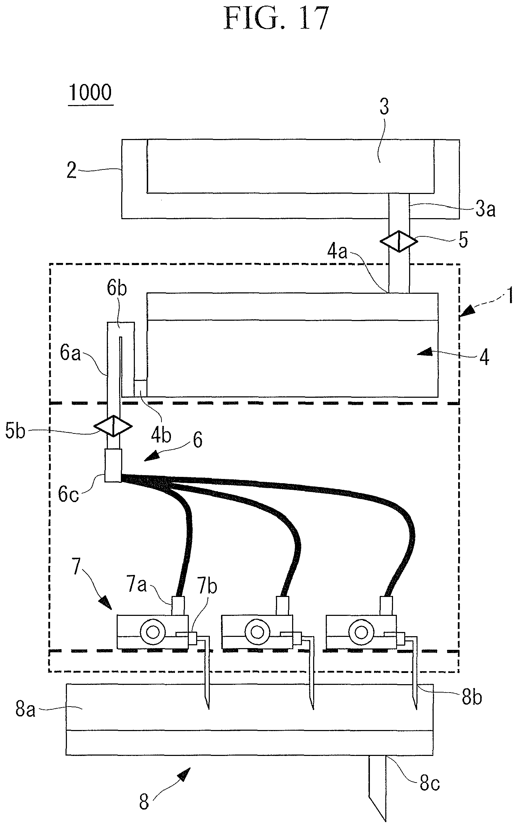

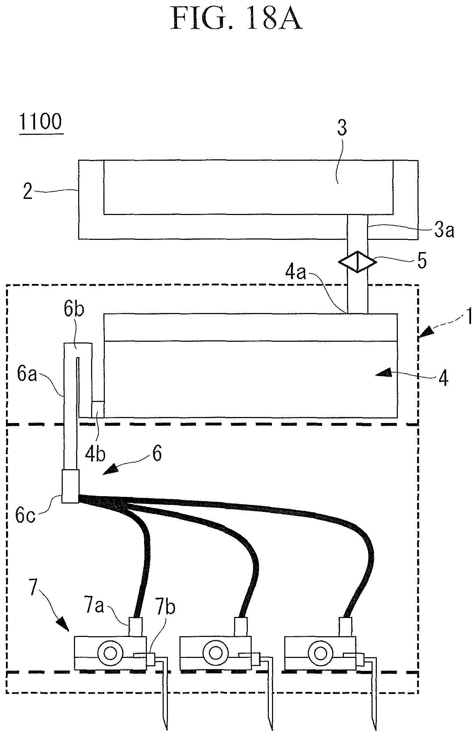

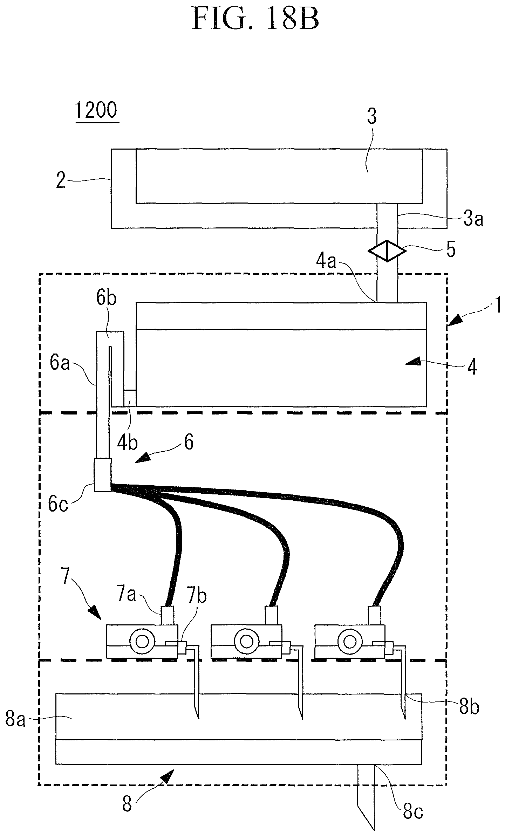

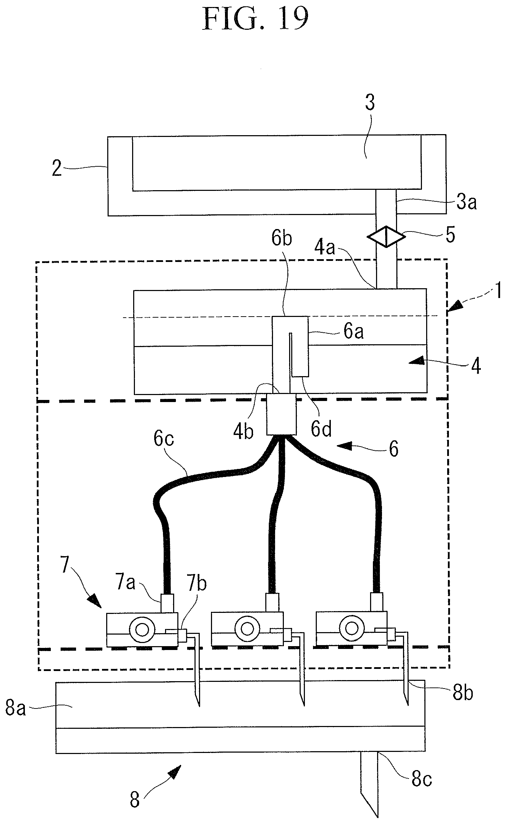

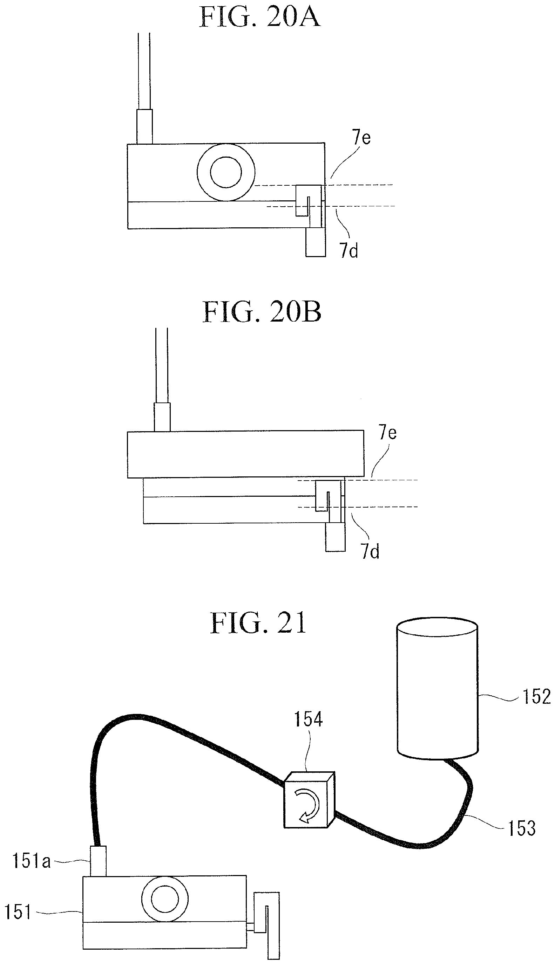

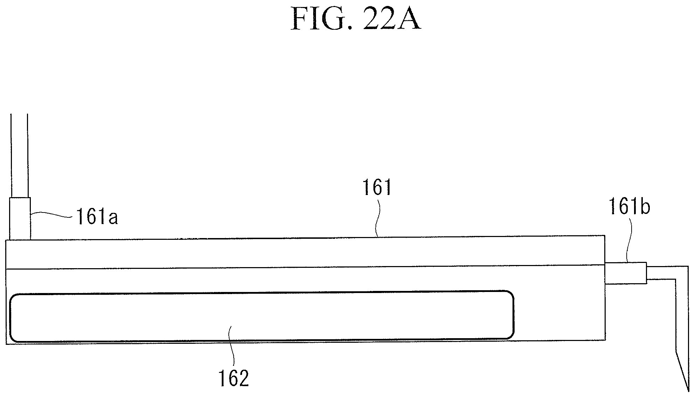

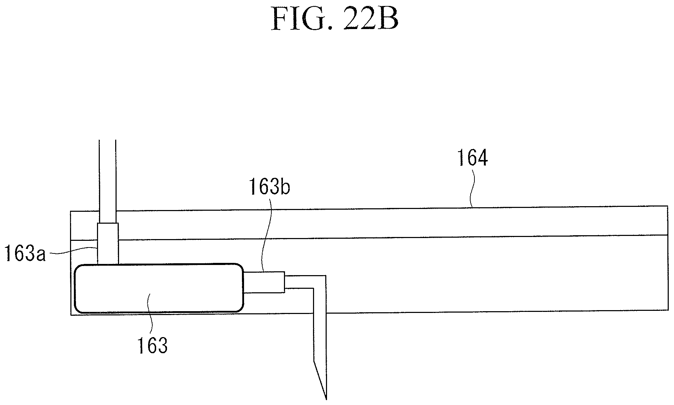

{Fig. 1A} Fig. 1A is an explanatory view showing, in outline, the configuration of a cell culture system according to a first embodiment of the present invention.
{Fig. 1B} Fig. 1B is an explanatory view showing, in outline, the configuration of a cell culture system according to the first embodiment of the present invention.
{Fig. 2A} Fig. 2A is an explanatory view showing, in outline, the configuration of an example supply-rate adjusting means of the present invention.
{Fig. 2B} Fig. 2B is an explanatory view showing, in outline, the configuration of an example supply-rate adjusting means of the present invention.
{Fig. 2C} Fig. 2C is an explanatory view showing, in outline, the configuration of an example supply-rate adjusting means of the present invention.
{Fig. 2D} Fig. 2D is an explanatory view showing, in outline, the configuration of an example supply-rate adjusting means of the present invention.
{Fig. 3A} Fig. 3A is an explanatory view showing, in outline, the configuration of an example siphon mechanism of the present invention.
{Fig. 3B} Fig. 3B is an explanatory view showing, in outline, the configuration of an example siphon mechanism of the present invention.
{Fig. 3C} Fig. 3C is an explanatory view showing, in outline, the configuration of an example siphon mechanism of the present invention.
{Fig. 3D} Fig. 3D is an explanatory view showing, in outline, the configuration of an example siphon mechanism of the present invention.
{Fig. 4A} Fig. 4A is an explanatory view showing, in outline, the configuration of a cell culture system according to a second embodiment of the present invention.
{Fig. 4B} Fig. 4B is an explanatory view showing, in outline, the configuration of a cell culture system according to the second embodiment of the present invention.
{Fig. 5A} Fig. 5A is an explanatory view showing, in outline, the configuration of an example supply gate of the present invention.
{Fig. 5B} Fig. 5B is an explanatory view showing, in outline, the configuration of an example supply gate of the present invention.
{Fig. 5C} Fig. 5C is an explanatory view showing, in outline, the configuration of an example supply gate of the present invention.
{Fig. 5D} Fig. 5D is an explanatory view showing, in outline, the configuration of an example supply gate of the present invention.
{Fig. 6A} Fig. 6A is an explanatory view showing, in outline, the configuration of an example supply gate of the present invention.
{Fig. 6B} Fig. 6B is an explanatory view showing, in outline, the configuration of an example supply gate of the present invention.
{Fig. 6C} Fig. 6C is an explanatory view showing, in outline, the configuration of an example supply gate of the present invention.
{Fig. 6D} Fig. 6D is an explanatory view showing, in outline, the configuration of an example supply gate of the present invention.
{Fig. 7} Fig. 7 is an explanatory view showing, in outline, the configuration of a cell culture system according to a third embodiment of the present invention.
{Fig. 8A} Fig. 8A is an explanatory view showing, in outline, the configuration of an example pressure supply means of the present invention.
{Fig. 8B} Fig. 8B is an explanatory view showing, in outline, the configuration of an example pressure supply means of the present invention.
{Fig. 8C} Fig. 8C is an explanatory view showing, in outline, the configuration of an example pressure supply means of the present invention.
{Fig. 9} Fig. 9 is an explanatory view showing, in outline, the configuration of a cell culture system according to a fourth embodiment of the present invention.
{Fig. 10} Fig. 10 is an explanatory view showing, in outline, the configuration of an example negative-pressure supply means of the present invention.
{Fig. 11} Fig. 11 is an explanatory view showing, in outline, the configuration of a cell culture system according to a fifth embodiment of the present invention.
{Fig. 12A} Fig. 12A is an explanatory view showing, in outline, the configuration of an example cell culture vessel that can be used in the present invention.
{Fig. 12B} Fig. 12B is an explanatory view showing, in outline, the configuration of an example cell culture vessel that can be used in the present invention.
{Fig. 12C} Fig. 12C is an explanatory view showing, in outline, the configuration of an example cell culture vessel that can be used in the present invention.
{Fig. 12D} Fig. 12D is an explanatory view showing, in outline, the configuration of an example cell culture vessel that can be used in the present invention.
{Fig. 13A} Fig. 13A is an explanatory view showing, in outline, the configuration of an example cell culture vessel that can be used in the present invention.
{Fig. 13B} Fig. 13B is an explanatory view showing, in outline, the configuration of an example cell culture vessel that can be used in the present invention.
{Fig. 13C} Fig. 13C is an explanatory view showing, in outline, the configuration of an example cell culture vessel that can be used in the present invention.
{Fig. 13D} Fig. 13D is an explanatory view showing, in outline, the configuration of an example cell culture vessel that can be used in the present invention.
{Fig. 14A} Fig. 14A is an explanatory view showing, in outline, the configuration of an example cell culture vessel that can be used in the present invention.
{Fig. 14B} Fig. 14B is an explanatory view showing, in outline, the configuration of an example cell culture vessel that can be used in the present invention.
{Fig. 15A} Fig. 15A is an explanatory view showing, in outline, the configuration of a modification of each of the embodiments of the present invention.
{Fig. 15B} Fig. 15B is an explanatory view showing, in outline, the configuration of a modification of each of the embodiments of the present invention.
{Fig. 16} Fig. 16 is an explanatory view showing, in outline, the configuration of a modification of each of the embodiments of the present invention.
{Fig. 17} Fig. 17 is an explanatory view showing, in outline, the configuration of a modification of each of the embodiments of the present invention.
{Fig. 18A} Fig. 18A is an explanatory view showing, in outline, the configuration of a modification of each of the embodiments of the present invention.
{Fig. 18B} Fig. 18B is an explanatory view showing, in outline, the configuration of a modification of each of the embodiments of the present invention.
{Fig. 19} Fig. 19 is an explanatory view showing, in outline, the configuration of a modification of each of the embodiments of the present invention.
{Fig. 20A} Fig. 20A is an explanatory view showing, in outline, the configuration of an example cell culture vessel that can be used in the present invention.
{Fig. 20B} Fig. 20B is an explanatory view showing, in outline, the configuration of an example cell culture vessel that can be used in the present invention.
{Fig. 21} Fig. 21 is an explanatory view showing, in outline, the configuration of a modification of each of the embodiments of the present invention.
{Fig. 22A} Fig. 22A is an explanatory view showing, in outline, the configuration of an example cell culture vessel that can be used in the present invention.
{Fig. 22B} Fig. 22B is an explanatory view showing, in outline, the configuration of an example cell culture vessel that can be used in the present invention.

### {Description of Embodiments}

Cell culture systems according to embodiments of the present invention will be described below with reference to the drawings.

### First Embodiment

A cell culture system 100 of this embodiment is a system that has a configuration shown in Figs. 1A and 1B and that replaces a culture medium in cell culture vessels (culture vessels) installed in an incubator.

A culture-medium storage means 3 is installed outside an incubator 1 and stores a culture medium (cell culture solution) therein. A temperature control means 2 is provided in order to maintain the temperature of the culture medium at an appropriate temperature for storage (for example, 4 °C).

The culture-medium storage means 3 is coupled to a temporary holding means 4 that is installed in the incubator 1, via a tubular member (tube or the like). Because the culture-medium storage means 3 is installed at a position higher in the direction of gravitational force than the temporary holding means 4, the culture medium in the culture-medium storage means 3 is gravitationally supplied to the temporary holding means 4 via the tubular member (tube or the like). The culture medium supplied to the temporary holding means 4 is held in a space (holding space) in the temporary holding means 4 and is heated to a temperature in the incubator (for example, 37 °C) that is an appropriate temperature for cell culturing.

The tubular member that connects the culture-medium storage means 3 and the temporary holding means 4 is provided with a supply-rate adjusting means 5, so that the flow rate of the culture medium flowing in the tubular member is controlled, thereby making it possible to adjust the supply rate of the culture medium from the culture-medium storage means 3 to the temporary holding means 4.

The culture medium heated, in the temporary holding means 4, to an appropriate temperature for cell culturing (for example, 37 °C) is supplied, in the incubator 1, to cell culture vessels 7 via a culture-medium supply means 6. The temporary holding means 4 is provided with: a supply port 4a through which the culture medium is supplied from the culture-medium holding means 3 via the tubular member; and a discharge port 4b through which the culture medium is discharged to the culture-medium supply means 6. Although the position where the supply port 4a is installed is arbitrarily decided, it is preferred to install the supply port 4a at an upper portion of the temporary holding means 4 to cause the supplied culture medium to be dripped into the inner space. Accordingly, backflow of the culture medium can be prevented, thereby making it possible to prevent the culture medium in the culture-medium storage means 3 from being contaminated (the occurrence of contamination). On the other hand, it is preferred that the installation site of the discharge port 4b be a place allowing the culture medium in the temporary holding means 4 to be completely discharged, i.e., a bottom surface or a lower portion on a side surface of the temporary holding means 4.

The culture-medium supply means 6 is provided with a siphon mechanism 6a (culture-medium intermittent supply mechanism). The siphon mechanism 6a is connected to the discharge port 4b of the temporary holding means 4 and has a structure in which a flow passage thereof is turned around, as shown in Figs. 1A and 1B. Specifically, the flow passage of the siphon mechanism 6a has a structure so as to extend from the discharge port 4b of the temporary holding means 4 upward in the direction of gravitational force to a predetermined height, turn around there, and extend downward in the direction of gravitational force. Here, the predetermined height that the flow passage of the siphon mechanism 6a reaches upward in the direction of gravitational force is set at a position lower than the maximum height in the direction of gravitational force that the culture medium held in the temporary holding means 4 can reach (hereinafter, this set height is referred to as "turn-around point 6b"). Accordingly, when the height of the culture medium in the temporary holding means 4 reaches the height of the turn-around point 6b or higher, the culture medium in the temporary holding means 4 starts to be discharged via the flow passage of the siphon mechanism 6a according to the siphon principle and keeps being discharged until the height of the culture medium in the temporary holding means 4 reaches the discharge port 4b of the temporary holding means 4. Then, the height of the culture medium in the temporary holding means 4 starts to rise due to the culture medium supplied from the culture-medium storage means 3, and, when the height of the culture medium in the temporary holding means 4 reaches a position higher than the turn-around point 6b of the flow passage of the siphon mechanism 6a, discharge occurs again. This process is repeated.

The flow passage of the siphon mechanism 6a extends upward in the direction of gravitational force from the discharge port 4b of the temporary culture-medium holding means 4 to the turn-around point 6b, passes through the turn-around point 6b, extends downward in the direction of gravitational force to reach a branching means 6c. From the branching means 6c, the flow passage is split into a plurality of tubular members (tubes or the like). The plurality of split tubular members are respectively connected to separate cell culture vessels 7 to supply the culture medium thereto.

The cell culture vessels 7 each have a supply port 7a to which the tubular member of the culture-medium supply means 6 is connected and a discharge port 7b through which the culture medium is discharged to the outside of the cell culture vessel. The discharge port 7b is disposed in a side surface of the cell culture vessel 7, and, when the amount of the culture medium (the height of the culture medium) in the cell culture vessel exceeds a certain value, the culture medium is discharged to a culture-medium discharge means 8 through the discharge port 7b. The amount of the culture medium held in the cell culture vessel 7 is determined by the installation height of the discharge port 7b. On the other hand, although the position where the supply port 7a is installed is arbitrarily decided, when it is installed as far away from the discharge port 7b as possible, the conversion efficiency of the culture medium can be increased. Furthermore, when the supply port 7a is installed at an upper portion of the cell culture vessel 7 to cause the supplied culture medium to be dripped into the cell culture vessel, backflow of the culture medium can be prevented, thus making it possible to prevent the culture medium in the culture-medium supply means 6, the temporary culture-medium holding means 4, and the culture-medium storage means 3, which are disposed at upstream sides of the system, from being contaminated (the occurrence of contamination).

The culture-medium discharge means 8 is provided with a waste-liquid holding means 8a, and the waste-liquid holding means 8a has: waste-liquid supply ports 8b to which tubular members extending from the cell culture vessels 7 are connected; and a waste-liquid discharge port 8c through which the culture medium is discharged to the outside of the waste-liquid holding means 8a. The waste-liquid supply ports 8b are installed in a top surface of the waste-liquid holding means 8a, and the culture medium supplied through each of the waste-liquid supply ports 8b is dripped into a space in the waste-liquid holding means 8a and is discharged through the waste-liquid discharge port 8c. In this way, when the culture medium is dripped into the space in the waste-liquid holding means 8a, backflow of the culture medium can be prevented, thus making it possible to prevent the insides of the cell culture vessels 7 from being contaminated (the occurrence of contamination).

Next, the supply-rate adjusting means 5 will be described.

The supply-rate adjusting means 5 is disposed in the tubular member (tube or the like) that connects the discharge port 3a of the culture-medium storage means 3 and the supply port 4a of the temporary holding means 4, applies an external force to deform the tubular member, thus reducing the cross-section area of a tubular-member lumen, thereby limiting the flow volume of the solution to suppress the flow rate. On the contrary, when the external force is released, the tubular member recovers to the original state due to the elastic force of the tubular member, thus making it possible to increase the flow rate. In this way, the supply-rate adjusting means 5 adjusts the flow rate of the solution flowing in the tubular member due to the magnitude of the external force applied to the tubular member. Figs. 2A to 2D show example ways of applying an external force to the tubular member by means of the supply-rate adjusting means 5. Fig. 2A shows an example in which a tubular member 20 is sandwiched between two platelike members 21. Fig. 2B shows an example in which the tubular member 20 passing through a through-hole 23 is sandwiched among a plurality of spherical (or columnar) members 22. Fig. 2C shows an example in which the tubular member 20 passing through a through-hole 25 is squeezed by using a shutter-like member 24. Fig. 2D shows an example in which the inner diameter of a through-hole 26 through which the tubular member 20 passes is reduced, thus deforming the tubular member. It is also possible to adopt a mechanism other than these examples as long as it is capable of deforming the tubular member by applying an external force.

Furthermore, a liquid feeding pump, such as a peristaltic pump, may be used as the supply-rate adjusting means 5. In this case, the culture-medium storage means 3 need not be disposed at a position higher than the temporary holding means 4 in the direction of gravitational force, thus increasing the degree of freedom in the installation site of the culture-medium storage means 3.

Next, an example procedure for replacing the culture medium by using the culture-medium replacement system 100 of this embodiment will be described.

A user of this system first sets the system in a state in which the supply rate of the culture medium is zero, i.e., in a state in which supply of the culture medium is stopped, by using the supply-rate adjusting means 5 and replenishes the culture-medium storage means 3 with the culture medium. The user prepares the cell culture vessels 7 in which the culture medium and cells are contained, connects, in the incubator, the supply ports 7a of the cell culture vessels 7 to the tubular members of the culture-medium supply means 6, and connects the discharge ports 7b of the cell culture vessels 7 to the waste-liquid supply ports 8b of the waste-liquid holding means 8a via the tubular members.

When culture-medium replacement is needed, the user first adjusts the supply-rate adjusting means 5 and sets the supply rate to an appropriate rate. When the state in which the supply rate is zero is released by means of the supply-rate adjusting means 5, the culture medium starts dripping into the temporary culture-medium holding means 4 due to the force of gravity. In adjusting the supply rate, the supply rate may be set to a rate determined in advance or may be adjusted to an appropriate supply rate while the user checks visually.

The culture medium is supplied to the temporary culture-medium holding means 4, and the culture medium level in the temporary culture-medium holding means 4 rises (Fig. 3A). When the culture medium level in the temporary culture-medium holding means 4 becomes higher than the height of the turn-around point 6b of the culture-medium supply means 6 (Fig. 3B), the culture medium in the temporary holding means 4 starts to be discharged via the culture-medium supply means 6 according to the siphon principle (Fig. 3C), keeps being discharged until the height of the culture medium in the temporary holding means 4 reaches the discharge port 4b of the temporary holding means 4, and stops discharging (Fig. 3D). Then, the height of the culture medium in the temporary holding means 4 starts to rise due to the culture medium supplied from the culture-medium storage means 3, and, when the height of the culture medium in the temporary holding means 4 reaches a position higher than the turn-around point 6b of the culture-medium supply means 6, discharge occurs again. This process is repeated.

The culture medium discharged from the temporary holding means 4 is supplied to the plurality of cell culture vessels 7 via the branching means 6c of the culture-medium supply means 6. When exceeding a specified amount in the cell culture vessels 7, the culture medium is discharged from the discharge ports 7b of the cell culture vessels 7 and is discharged to the outside of the incubator via the waste-liquid holding means 8a. Accordingly, the old culture medium in the cell culture vessels 7 is replaced with a new culture medium, thereby making it possible to reduce the rate of deterioration of the culture medium.

In this embodiment, as the siphon mechanism 6a, a tubular member (tube or the like) may form a flow passage, as shown in Fig. 1B. Specifically, the siphon mechanism 6a may have a structure in which the tubular member connected to the discharge port 4b of the temporary holding means 4 extends upward in the direction of gravitational force to the height of the turn-around point 6b, turns around there, and extends downward in the direction of gravitational force. The tubular member that has passed through the turn-around point 6b and has extended downward reaches the branching means 6c, and the plurality of tubular members split from the branching means 6c are respectively connected to the separate cell culture vessels 7 to supply the culture medium thereto.

### Second Embodiment

A cell culture system 300 of this embodiment is a system that has a configuration shown in Figs. 4A and 4B and that replaces the culture medium in the cell culture vessels (culture vessels) installed in the incubator.

The culture-medium storage means 3 is installed outside the incubator 1 and stores the culture medium (cell culture solution) therein. The temperature control means 2 is provided in order to maintain the temperature of the culture medium to an appropriate temperature for storage (for example, 4 °C).

The culture-medium storage means 3 is coupled to the temporary holding means 4, which is installed in the incubator 1, via the tubular member (tube or the like). Because the culture-medium storage means 3 is installed at a position higher in the direction of gravitational force than the temporary holding means 4, the culture medium in the culture-medium storage means 3 is gravitationally supplied to the temporary holding means 4 via the tubular member (tube or the like). The culture medium supplied to the temporary holding means 4 is heated to a temperature in the incubator (for example, 37 °C) that is an appropriate temperature for cell culturing.

The tubular member that connects the culture-medium storage means 3 and the temporary holding means 4 is provided with the supply-rate adjusting means 5, and the flow rate of the culture medium flowing in the tubular member is controlled, thereby making it possible to adjust the supply rate of the culture medium from the culture-medium storage means 3 to the temporary holding means 4.

The culture medium heated, in the temporary holding means 4, to an appropriate temperature for cell culturing (for example, 37 °C) is supplied, in the incubator 1, to the cell culture vessels 7 via the culture-medium supply means 6. The temporary holding means 4 is provided with: the supply port 4a, through which the culture medium is supplied from the culture-medium holding means 3 via the tubular member; and the discharge port 4b, through which the culture medium is discharged to the culture-medium supply means 6. Although the position where the supply port 4a is installed is arbitrarily decided, it is preferred to install the supply port 4a at an upper portion of the temporary holding means 4 to cause the supplied culture medium to be dripped into the inner space. Accordingly, backflow of the culture medium can be prevented, thereby making it possible to prevent the culture medium in the culture-medium storage means 3 from being contaminated (the occurrence of contamination). On the other hand, it is preferred that the installation site of the discharge port 4b be a place in the temporary holding means 4 for allowing the culture medium therein to be completely discharged.

The culture-medium supply means 6 is provided with a supply gate 6d (culture-medium intermittent supply mechanism) at a connection part with the temporary culture-medium holding means 4. The supply gate 6d is a means that is capable of opening and closing the flow passage according to the amount of the culture medium in the temporary holding means 4, that opens the gate to discharge the culture medium from the temporary holding means 4 when the amount of the culture medium in the temporary culture-medium holding means 4 exceeds a predetermined amount, and that closes the gate to block discharge of the culture medium from the temporary holding means 4 when the culture medium in the temporary holding means 4 decreases to the predetermined amount. The supply gate 6d is connected to the cell culture vessels 7 via the tubular members.

When reaching the branching means 6c, the tubular member of the culture-medium supply means 6 extending from the supply gate 6d is split into a plurality of tubular members. The plurality of split tubular members are respectively connected to the separate cell culture vessels 7 to supply the culture medium thereto.

The cell culture vessels 7 each have the supply port 7a, to which the tubular member of the culture-medium supply means 6 is connected, and the discharge port 7b, through which the culture medium is discharged to the outside of the cell culture vessel. The discharge port 7b is disposed in a side surface of the cell culture vessel 7, and, when the amount of the culture medium (the height of the culture medium) in the cell culture vessel exceeds a certain value, the culture medium is discharged to the culture-medium discharge means 8 via the discharge port 7b. The amount of the culture medium held in the cell culture vessel is determined by the installation height of the discharge port 7b. On the other hand, although the position where the supply port 7a is installed is arbitrarily decided, when it is installed as far away from the discharge port 7b as possible, the conversion efficiency of the culture medium can be increased. Furthermore, when the supply port 7a is installed at an upper portion of the cell culture vessel 7 to cause the supplied culture medium to be dripped into the cell culture vessel 7, backflow of the culture medium can be prevented, thus making it possible to prevent the culture medium in the culture-medium supply means 6, the temporary culture-medium holding means 4, and the culture-medium storage means 3, which are disposed at upstream sides of the system, from being contaminated (the occurrence of contamination).

The culture-medium discharge means 8 is provided with the waste-liquid holding means 8a, and the waste-liquid holding means 8a has: the waste-liquid supply ports 8b, to which the tubular members extending from the cell culture vessels 7 are connected; and the waste-liquid discharge port 8c, through which the culture medium is discharged to the outside of the waste-liquid holding means 8a. The waste-liquid supply ports 8b are installed in the top surface of the waste-liquid holding means 8a, and the culture medium supplied through each of the waste-liquid supply ports 8b is dripped into a space in the waste-liquid holding means 8a and is discharged through the waste-liquid discharge port 8c. In this way, when the culture medium is dripped into the space in the waste-liquid holding means 8a, backflow of the culture medium can be prevented, thus making it possible to prevent the insides of the cell culture vessels 7 from being contaminated (the occurrence of contamination).

The supply-rate adjusting means 5 is the same as that in the first embodiment.

Next, the supply gate 6d will be described.

The supply gate 6d is a means capable of opening and closing the flow passage according to the amount of the culture medium in the temporary holding means 4 and has a configuration shown in Figs. 5A to 5D, for example.

Figs. 5A and 5B show examples in which the flow passage is blocked by using a valve 31. When no force is applied, the valve 31 is located at a position blocking the flow passage, due to the elastic force thereof. When the pressure from the culture medium in the temporary holding means 4 exceeds a threshold, the valve 31 opens, thus opening the flow passage and discharging the culture medium from the temporary holding means 4. When the culture medium is discharged, and the pressure from the culture medium on the valve 31 starts to decrease and becomes lower than the threshold, the valve 31 returns to the position blocking the flow passage, due to the elastic force thereof, thereby blocking discharge of the culture medium from the temporary holding means 4. Then, when the amount of the culture medium in the temporary holding means 4 increases, and the pressure from the culture medium on the valve 31 exceeds the threshold, the valve 31 is pressed again, thus opening the flow passage. This process is repeated. Here, Fig. 5A shows a form in which a ring member 32 prevents the valve 31 from falling over in the opposite direction of the flow passage, and Fig. 5B shows a form in which the flow passage has a structure so as to expand in the flow direction, thus preventing the valve 31 from falling over in the opposite direction of the flow passage.

Figs. 5C and 5D show examples in which the flow passage is blocked by using a ball 33. When no force is applied, the ball 33 is located at a position blocking the flow passage, by being pressed by an elastic member 34 (for example, a spring) against the ring member 32. When the pressure from the culture medium in the temporary holding means 4 exceeds the threshold, the ball 33 is pressed in the flow direction, thus opening the flow passage and discharging the culture medium from the temporary holding means 4. When the culture medium is discharged, and the pressure from culture medium on the ball 33 decreases and becomes lower than the threshold, the ball 33 returns to the position blocking the flow passage, due to the elastic force of the elastic member 34, thereby blocking discharge of the culture medium from the temporary holding means 4. Then, when the amount of the culture medium in the temporary holding means 4 increases, and the pressure from the culture medium on the ball 33 exceeds the threshold, the ball 33 is pressed again, thus opening the flow passage. This process is repeated. Here, Fig. 5C shows a form in which the ball 33 is pressed against the ring member 32, thus blocking the flow passage, and Fig. 5D shows a form in which the flow passage has a structure so as to expand in the direction of the flow passage, and the ball 33 is pressed against a narrowed portion of the flow passage, thus blocking the flow passage.

Next, an example procedure for replacing the culture medium by using the culture-medium replacement system 300 of this embodiment will be described.

A user of this system first sets the system in a state in which the supply rate of the culture medium is zero, i.e., in a state in which supply of the culture medium is stopped, by using the supply-rate adjusting means 5 and replenishes the culture-medium storage means 3 with the culture medium. The user prepares the cell culture vessels 7 in which the culture medium and cells are contained, connects, in the incubator, the supply ports 7a of the cell culture vessels 7 to the tubular members of the culture-medium supply means 6, and connects the discharge ports 7b of the cell culture vessels 7 to the waste-liquid supply ports 8b of the waste-liquid holding means 8a via the tubular members.

When culture-medium replacement is needed, the user first adjusts the supply-rate adjusting means 5 and sets the supply rate to an appropriate rate. When the state in which the supply rate is zero is released by means of the supply-rate adjusting means 5, the culture medium starts dripping into the temporary culture-medium holding means 4 due to the force of gravity. In adjusting the supply rate, the supply rate may be set to a rate determined in advance or may be adjusted to an appropriate supply rate while the user checks visually.

The culture medium is supplied to the temporary culture-medium holding means 4, and the amount of the culture medium in the temporary culture-medium holding means 4 increases. When the pressure from the culture medium in the temporary culture-medium holding means 4 on the supply gate 6d exceeds the threshold, the gate opens, and the culture medium in the temporary holding means 4 starts to be discharged via the culture-medium supply means 6, keeps being discharged until the pressure from the culture medium in the temporary culture-medium holding means 4 on the supply gate 6d decreases to the threshold, and stops being discharged when the pressure decreases to the threshold. Then, when the amount of the culture medium in the temporary holding means 4 starts to increase due to the culture medium supplied from the culture-medium storage means 3, and the pressure from the culture medium in the temporary culture-medium holding means 4 on the supply gate 4d exceeds the threshold, discharge occurs again. This process is repeated.

The culture medium discharged from the temporary holding means 4 is supplied to the plurality of cell culture vessels 7 via the branching means 6c of the culture-medium supply means 6. When exceeding a specified amount in the cell culture vessels 7, the culture medium is discharged from the discharge ports 7b of the cell culture vessels 7 and is discharged to the outside of the incubator via the waste-liquid holding means 8a. Accordingly, the old culture medium in the cell culture vessels 7 is replaced with a new culture medium, thereby making it possible to reduce the rate of deterioration of the culture medium.

Figs. 6A to 6D show other forms of the supply gate of this embodiment.

Figs. 6A and 6B show examples in which the flow passage is blocked by using a valve 41. The valve 41 is connected to a float 42 via a string-like member 45. The float 42 has a structure allowing it to float on the top surface of the culture medium in the temporary holding means 4. When the amount of the culture medium in the temporary holding means 4 increases, the height of the top surface of the culture medium rises, and the float 42 rises accordingly. When the float 42 rises, tension occurs in the string-like member 45, and the valve 41 receives an upward force from the string-like member 45. When the height of the top surface of the culture medium exceeds the threshold, the valve 41 opens, thus opening the flow passage and discharging the culture medium from the temporary holding means 4. When the culture medium is discharged, the top surface of the culture medium in the temporary holding means 4 starts to fall, the float 42 also falls, thus reducing the tension of the string-like member 45, and the valve 41 returns to a position blocking the flow passage, due to the elastic force thereof (or the force of gravity), thus blocking discharge of the culture medium from the temporary holding means 4. Then, when the amount of the culture medium in the temporary holding means 4 increases, and the height of the top surface of the culture medium exceeds the threshold, the valve 41 is raised up again, thus opening the flow passage. This process is repeated. Here, Fig. 6A shows a form in which a ring member 43 prevents the valve 41 from falling over in the opposite direction of the flow passage, and Fig. 6B shows a form in which the flow passage has a structure so as to be tapered in the flow direction, thus preventing the valve 41 from falling over in the opposite direction of the flow passage.

Figs. 6C and 6D show examples in which the flow passage is blocked by using a ball 44 instead of the valve 41. Fig. 6C shows a form in which the ball 44 is pressed against the ring member 43, thus blocking the flow passage, and Fig. 6D shows a form in which the flow passage has a structure so as to be tapered in the direction of the flow passage, and the ball 44 is pressed against a narrowed portion of the flow passage, thus blocking the flow passage.

### Third Embodiment

A cell culture system 500 of this embodiment differs from those of the above-described embodiments in that a pressure applying means 51 that applies a pressure to the culture medium in the culture-medium storage means 3 is provided, as shown in Fig. 7. The other components are the same as those in the above-described embodiments. Although Fig. 7 shows an example corresponding to the first embodiment, the same applies to the other embodiment.

As the pressure applying means 51, a means for pumping gas into the culture-medium storage means 3 by means of a pump can be used, for example. Accordingly, the pressure of an air space in the culture-medium storage means 3 is increased, thus making it possible to apply an external pressure to the culture medium. It is preferred that gas to be pumped into the culture-medium storage means 3 be sterilized. As shown in Fig. 8A, it is also possible to pump gas (or liquid) into a saclike member 61, thus applying a pressure to the culture medium with the sac that has been increased in volume, or, as shown in Fig. 8B, it is also possible to isolate the culture medium from an air space 63 by using a movable isolation member 62 while securing airtightness, to pump gas (or liquid) into the air space 63, and to apply a pressure to the culture medium by means of the isolation member 62. Accordingly, the risk of contamination of the culture medium can be reduced. Note that, as shown in Fig. 8C, in a form in which an isolation member shown in Fig. 8C is used, a weight 65 having a predetermined weight may be placed on an upper portion of an isolation member 64, instead of pumping gas (or liquid), thereby applying a certain pressure to the isolation member 64. The culture-medium holding means and the isolation member have structures like a tube and a pusher (plunger) of a syringe, respectively, and the isolation member, which serves as a pusher (plunger), may be mechanically moved to apply a pressure to the culture medium in the culture-medium holding means. In this case, the movement of the isolation member may be controlled by a control unit in a wired or wireless manner.

Furthermore, it is also possible to adopt a form in which the culture-medium holding means has a column-shaped structure, the isolation member has a disk-shaped structure that fits in the inner wall of the column-shaped structure while maintaining airtightness, and the inner wall of the column-shaped structure and the circumference of the disk-shaped structure have screw structures to be engaged with each other. In this case, when the isolation member is attached to the inside of the culture-medium holding means and is rotated in the disk circumferential direction, the isolation member is moved in the height direction of the column structure, thus making it possible to apply a pressure to the culture medium in the culture-medium holding means (or remove the pressure therefrom). The rotation of the isolation member may be mechanically performed, and the rotation of the isolation member may be controlled by the control unit in a wired or wireless manner.

Here, it is preferred that the pressure applying means 51 be controlled such that the pressure in the culture-medium storage means 3 does not increase to a certain level or more.

Note that, in this embodiment, the culture-medium storage means 3 is not necessarily disposed at a higher position than the temporary holding means 4. Furthermore, the temporary holding means 4 is not necessarily disposed at a higher position than the culture-medium supply means 6.

### Fourth Embodiment

As shown in Fig. 9, a cell culture system 600 of this embodiment differs from those of the above-described embodiments in that a negative-pressure supply means 71 that applies a negative pressure to the discharge ports 7b of the cell culture vessels 7 is provided. The other components are the same as those in the above-described embodiments. Although Fig. 9 shows an example corresponding to the first embodiment, the same applies to the other embodiments.

As the negative-pressure supply means 71, it is possible to adopt a means that is provided with a pump 81 and a waste-liquid container 82 shown in Fig. 10. A suction port 83 of the negative-pressure supply means 71 is connected to the waste-liquid discharge port 8b of the waste-liquid holding means 8a to make the inside of the waste-liquid holding means 8a have a negative pressure and to make the discharge ports 7b of the cell culture vessels 7 have a negative pressure, thereby making it possible to suction the culture medium from the cell culture vessels 7.

As the pump of the negative-pressure supply means, a liquid feeding pump, such as a peristaltic pump, may be used. In this case, the liquid feeding pump may be disposed on a tubular member of the suction port 83.

### Fifth Embodiment

As shown in Fig. 11, in a cell culture system 700 of this embodiment, the supply-rate adjusting means 5 can be remotely controlled in a wired or wireless manner by using a control unit 19 that is provided outside the incubator, in each of the above-described embodiments. The other components are the same as those in the above-described embodiments. Although Fig. 11 shows an example corresponding to the first embodiment, the same applies to the other embodiments.

In this embodiment, the supply-rate adjusting means 5 can exchange information with the control unit 19, which is provided outside the incubator, in a wired or wireless manner, and the flow rate of a solution, such as a culture medium, flowing in the tubular member (tube or the like) can be remotely controlled.

When culture-medium replacement is needed, the user remotely adjusts the supply-rate adjusting means 5 by using the control unit 19 and sets the supply rate to an appropriate rate. In adjusting the supply rate, the supply rate may be set to a rate determined in advance or may be adjusted to an appropriate supply rate while monitoring a drip rate by using a monitoring system (not shown).

According to this embodiment, the user can start to remotely replace the culture medium or can remotely change the supply rate, at arbitrary timing during the cell culturing. For example, when used with a system (not shown) capable of remotely monitoring the state of the cells, it is possible to start to remotely replace the culture medium or remotely change the supply rate at arbitrary timing during the cell culturing in accordance with the state of the cells. By doing so, because the user can work without entering a work space, it is possible to save the trouble of having to change into disposable work clothes and the costs thereof and to reduce the risk of contamination of the cell culture system by bacteria etc.

The control unit 19 of this embodiment may be able to exchange information with the temperature control means 2, the pressure applying means 51, and the negative-pressure supply means 71, which are provided in the above-described embodiments, in a wired or wireless manner and may be able to remotely control them. Accordingly, it is possible to improve the user's remote work efficiency.

In the above-described embodiments, a description has been given of a case in which the discharge port of each cell culture vessel has a form shown in Fig. 12A, i.e., a form in which the discharge port 7b is disposed on the side surface of the cell culture vessel 7, and the culture medium is discharged to the culture-medium discharge means 8 through the discharge port 7b when the amount of the culture medium (the height of the culture medium) in the cell culture vessel exceeds a certain value; however, the discharge port of the cell culture vessel is not limited thereto and may have forms shown Figs. 12A, 12B, 12C, and 12D, for example.

Figs. 12B and 12C show forms that are each provided with a mechanism (discharge siphon mechanism 7d) similar to the siphon mechanism 6a of the culture-medium supply means 6. Specifically, the discharge siphon mechanism 7d has a structure in which the flow passage extends upward in the direction of gravitational force from the discharge port 7b of the cell culture vessel to a predetermined height, turns around there, and extends downward in the direction of gravitational force. The predetermined height to which the flow passage extends upward in the direction of gravitational force is set at a position lower than the maximum height in the direction of gravitational force that the culture medium held in the cell culture vessel can reach (hereinafter, this set height is referred to as "turn-around point 7e"). Accordingly, when the height of the culture medium in the cell culture vessel reaches the height of the turn-around point 7e or higher, the culture medium in the cell culture vessel starts to be discharged via the flow passage of the discharge siphon mechanism 7d according to the siphon principle, and keeps being discharged until the height of the culture medium in the cell culture vessel reaches the discharge port 7b of the cell culture vessel.

Then, the culture medium is supplied from the culture-medium supply means 6, and, when the height of the culture medium in the cell culture vessel reaches a position higher than the turn-around point 7e of the flow passage of the discharge siphon mechanism 7d, discharge occurs again. This process is repeated. Here, the discharge siphon mechanism 7d functions as a culture-medium intermittent discharge mechanism.

Fig. 12D shows a form that can be applied to the system that is provided with the negative-pressure supply means 71 for applying a negative pressure to the cell culture vessels 7. In this form, the tubular member of the culture-medium discharge means 8 passes through the discharge port 7b of the cell culture vessel and extends to a predetermined height (7f) from the bottom surface of the cell culture vessel. Accordingly, when the height of the culture medium in the cell culture vessel reaches the predetermined height (7f) or higher, the culture medium corresponding to a volume above the predetermined height (7f) is discharged to the outside of the cell culture vessel due to a negative pressure in the tubular member of the culture-medium discharge means 8.

Then, the culture medium is supplied from the culture-medium supply means 6, and, when the height of the culture medium in the cell culture vessel reaches the predetermined height (7f), discharge occurs again. This process is repeated. The predetermined height (7f) is set according to the type of cells to be cultured and the culture conditions.

Although Figs. 12A to 12D show flask-like cell culture vessels, petri-dish-like cell culture vessels may be used, as shown in Figs. 13A to 13D. In Figs. 13A to 13D, identical reference signs are assigned to the portions corresponding to those in Figs. 12A to 12D.

In the above-described embodiments, although the flask-like culture vessels are shown in the figures as the cell culture vessels to be used, it is also possible to use petri-dish-like culture vessels, as shown in Figs. 13A to 13D. Furthermore, it is also possible to use a saclike cell culture bag that is provided with a supply port through which a solution is supplied and a discharge port through which the solution is discharged. Furthermore, as shown in Figs. 14A and 14B, it is also possible to use a cell culture vessel that has thresholds forming a flow passage. This vessel is used, and a supply port 141 and a discharge port 142 are disposed at distant positions along the flow passage, thus improving culture-medium replacement efficiency.

In the forms that are provided with the siphon mechanisms of the above-described embodiments, although a case in which a single siphon mechanism of the culture-medium supply means is provided has been illustrated, it is also possible to provide a plurality of siphon mechanisms, as shown in Fig. 15A. In this case, when the connection portions between the respective siphon mechanisms and the temporary holding means 4 are disposed at different heights in the direction of gravitational force, if the siphon mechanism that is disposed lower in the direction of gravitational force does not work, the siphon mechanism that is disposed higher in the direction of gravitational force can provide a backup. Although Fig. 15A shows an example corresponding to the first embodiment, the same applies to the other embodiments.

In the above-described embodiments, as shown in Fig. 15B, the temporary holding means 4 may be provided with a backup discharge port 4c. When the backup discharge port 4c is connected to the culture-medium supply means 6 (for example, the branching means 6c) via a tubular member, if the siphon mechanism 6a does not work, and the culture medium in the temporary holding means 4 reaches the backup discharge port 4c, the culture medium can be made to flow out to the culture-medium supply means. The tubular member from the backup discharge port 4c may be connected, not to the culture-medium supply means 6, for example, but to the culture-medium discharge means 8 or to the culture-medium storage means 3. Although Fig. 15B shows an example corresponding to the first embodiment, the same applies to the other embodiments.

The above-described embodiments show a form in which the culture-medium storage means 3 is coupled to the temporary holding means 4 via the tubular member (tube or the like), the culture-medium storage means 3 is disposed at a position higher in the direction of gravitational force than the temporary holding means 4, and thus, the culture medium in the culture-medium storage means 3 is gravitationally supplied to the temporary holding means 4 via the tubular member (tube or the like); however, for example, as shown in Fig. 16, it is also possible to provide, as the supply-rate adjusting means, a liquid feeding pump 5a, such as a peristaltic pump, in the tubular member (tube or the like) connecting the culture-medium storage means and the temporary holding means, and to supply the culture medium from the culture-medium storage means 3 to the temporary holding means 4. In this case, the culture-medium holding means 3 is not necessarily disposed at a position higher in the direction of gravitational force than the temporary holding means 4, thus increasing the degree of freedom in the installation site of the culture-medium holding means 3. In this case, the liquid feeding pump 5a, such as a peristaltic pump, may be remotely controlled by a control means. The remote control may be performed in a wired or wireless manner. Although Fig. 16 shows an example corresponding to the first embodiment, the same applies to the other embodiments.

In the above-described embodiments, for example, as shown in Fig. 17, the culture-medium supply means 6 may be provided with a supply-rate adjusting means 5b. It is preferred that the supply-rate adjusting means 5b be installed in the flow passage at an upstream side of the branching means 6c. Accordingly, the supply rate of the culture medium supplied to the cell culture vessels can be adjusted according to the culture conditions, thus improving the convenience. The supply-rate adjusting means 5b may have the same configuration as the supply-rate adjusting means 5, which is disposed in the tubular member (tube or the like) connecting the culture-medium storage means 3 and the temporary holding means. Furthermore, as in the supply-rate adjusting means 5, the supply-rate adjusting means 5b may be remotely controlled by the control means in a wired or wireless manner.

In the above-described embodiments, an example case in which a waste liquid discharged through the discharge ports of the culture vessels is discharged via the waste-liquid holding means has been illustrated; however, for example, as shown in Fig. 18A, the waste liquid may be directly discharged through the discharge ports of the cell culture vessels, without providing the waste-liquid holding means. Although Fig. 18 shows an example corresponding to the first embodiment, the same applies to the other embodiments.

In this case, in the third embodiment, the suction port of the negative-pressure supply means may be connected to the discharge ports of the cell culture vessels.

In the above-described embodiments, although a form in which a single culture-medium holding means and a single temporary holding means are provided has been illustrated, a plurality of culture-medium holding means and a plurality of temporary holding means may be provided. In this case, multiple types of culture media can be supplied to the cell culture vessels.

In the above-described embodiments, although a form in which connections are made from a single culture-medium supply means to a plurality of cell culture vessels has been illustrated, it is also possible to adopt a form in which a connection may be made from a single culture-medium supply means to a single cell culture vessel.

In the above-described embodiments, a culture-medium temperature monitoring means that monitors the culture-medium temperature may be provided in the temporary holding means. At this time, information of the culture-medium temperature monitored by the culture-medium temperature monitoring means may be remotely sent to the control unit. Thus, it is possible to prevent the culture medium at a temperature that is unsuitable for cell culturing from being accidentally supplied.

Furthermore, the amount of the culture medium held in each cell culture vessel is set to the lowest possible amount, thereby making it possible to improve the culture-medium replacement efficiency. The amount of the culture medium held in the cell culture vessel may be optimized depending on the type of cells to be cultured.

In the above-described embodiments, the control unit, which is provided outside the incubator, may be able to exchange information in a wired or wireless manner with the temperature control means, the pressure applying means, the negative-pressure supply means, and the liquid feeding pump, which are provided in the above-described embodiments, and may be able to remotely control them. Accordingly, it is possible to improve the user's remote work efficiency.

In the above-described embodiments, in the form in which the user remotely controls culture-medium replacement by using the control unit, the control unit may remotely control culture-medium replacement on the basis of a schedule (program) set in advance by the user.

In the above-described embodiments, although a form in which the temporary holding means is disposed inside the incubator has been illustrated, the temporary holding means may be disposed outside the incubator. In that case, it is preferred to provide a temperature control means for maintaining the solution in the temporary holding means at an appropriate temperature for cell culturing (for example, 37 °C).

Furthermore, in the above-described embodiments, although a form in which the waste-liquid holding means is disposed outside the incubator has been illustrated, for example, as shown in Fig. 18B, the waste-liquid holding means may be disposed inside the incubator.

A PC can be shown as an example of the control unit of the present invention, and the PC can perform control performed by the control unit in the above-described embodiments.

Specifically, the control unit is, for example, a PC that has a CPU and a memory, and, the CPU executes a control program written in the memory, thereby realizing the functions as the control unit.

In the present invention, the temperature of the culture medium in the culture-medium storage means 3 may be set at an appropriate temperature according to the culture conditions. The culture-medium temperature may be maintained at 37 °C by means of the temperature control means 2. Furthermore, the culture-medium storage means 3 may be disposed inside the incubator, and, in that case, if the culture-medium temperature is maintained at 37 °C, the temperature control means 2 may be omitted.

In the present invention, the culture-medium storage means and the temporary culture-medium holding means are means capable of holding the culture medium or another solution (for example, a cleaning solution or the like) and serve as solution holding means. Each of the culture-medium storage means and the temporary culture-medium holding means serves as a solution holding means, and both of the means, as a unit, also serve as a solution holding means.

The present invention can provide a cell culture system including:
a culture-medium storage means that holds a culture medium for culturing cells;
a temporary holding means that is coupled to the culture-medium storage means and that is provided with a holding space for temporarily holding the culture medium supplied from the culture-medium storage means and a discharge port for discharging the culture medium;
a culture-medium supply means that is coupled to the discharge port of the temporary holding means and that supplies the culture medium discharged from the temporary holding means, to a cell culture vessel; and
a culture-medium discharge means that discharges the culture medium from the cell culture vessel,
wherein the culture-medium supply means is provided with a culture-medium intermittent supply mechanism that intermittently supplies the culture medium discharged from the temporary holding means, to the cell culture vessel in a predetermined cycle.

The present invention can provide a cell culture system including:
a culture-medium storage means that holds a culture medium for culturing cells;
a temporary holding means that is coupled to the culture-medium storage means and that is provided with a holding space for temporarily holding the culture medium supplied from the culture-medium storage means and a discharge port for discharging the culture medium;
a culture-medium supply means that is coupled to the discharge port of the temporary holding means and that supplies the culture medium discharged from the temporary holding means, to a cell culture vessel; and
a culture-medium discharge means that discharges the culture medium from the cell culture vessel,
wherein the culture-medium discharge means is provided with a culture-medium intermittent discharge mechanism that intermittently discharges the culture medium in the cell culture vessel to the outside in a predetermined cycle.

In the above-described first embodiment and modifications thereof, although a form in which the siphon mechanism 6a is disposed outside the temporary holding means 4 has been illustrated, for example, as shown in Fig. 19, the siphon mechanism 6a may be disposed inside the temporary holding means 4. In this case, a tubular member that forms the flow passage of the siphon mechanism 6a has a structure so as to extend from the discharge port 4b of the temporary holding means 4 toward the inside of the temporary holding means 4 upward in the direction of gravitational force to a predetermined height, turn around there, and extend downward in the direction of gravitational force. An end of the tubular member extending downward in the direction of gravitational force has a structure so as to have an opening 6d in the vicinity of the bottom surface of the temporary holding means 4 with a gap therebetween. Here, the predetermined height that the flow passage of the siphon mechanism 6a reaches upward in the direction of gravitational force is set at a position lower than the maximum height in the direction of gravitational force that the culture medium held in the temporary holding means 4 can reach (hereinafter, this set height is referred to as "turn-around point 6b").

Accordingly, when the height of the culture medium in the temporary holding means 4 reaches the height of the turn-around point 6b or higher, the culture medium in the temporary holding means 4 starts to be discharged from the discharge port 4b via the flow passage of the siphon mechanism 6a according to the siphon principle and keeps being discharged until the height of the culture medium in the temporary holding means 4 reaches the opening 6d of the siphon mechanism 6a. Then, the height of the culture medium in the temporary holding means 4 starts to rise due to the culture medium supplied from the culture-medium storage means 3, and, when the height of the culture medium in the temporary holding means 4 reaches a position higher than the turn-around point 6b of the flow passage of the siphon mechanism 6a, discharge occurs again. This process is repeated.

Specifically, the siphon mechanism 6a of the present invention is a mechanism that is provided with a flow passage capable of intermittently supplying the culture medium from the temporary holding means 4 to the cell culture vessel according to the siphon principle.

According to the present invention, it is possible to provide a cell culture vessel including: a supply port for supplying a culture medium; a discharge port for discharging the culture medium; and a tubular member (discharge siphon mechanism) that is connected to the discharge port, wherein the tubular member forms a flow passage that allows the culture medium in the cell culture vessel to be intermittently discharged according to the siphon principle.

For example, it is possible to provide a cell culture vessel 7, such as those shown in Figs. 12B, 12C, 13B, and 13C. Specifically, the cell culture vessel 7 includes: a supply port 7a for supplying a culture medium; a discharge port 7b for discharging the culture medium; and a tubular member (discharge siphon mechanism) that is connected to the discharge port 7b, wherein the discharge port 7b is disposed in a vessel side surface at the position of a predetermined height from the vessel bottom surface; and the tubular member forms a flow passage that passes through a position that is higher in the direction of gravitational force than the discharge port 7b and that is lower in the direction of gravitational force than the maximum height of the space in the vessel.

Furthermore, it is possible to provide a cell culture vessel 7 such as those shown in Figs. 20A and 20B. Specifically, the cell culture vessel 7 includes: a supply port 7a for supplying a culture medium; a discharge port 7b for discharging the culture medium; and a tubular member (discharge siphon mechanism) that is connected to the discharge port 7b, wherein the tubular member is disposed inside the cell culture vessel 7 and forms a flow passage that passes through a position that is higher in the direction of gravitational force than the discharge port 7b and that is lower in the direction of gravitational force than the maximum height of the space in the vessel; and an end of the tubular member that is opposite from an end thereof that is connected to the discharge port 7b has an opening in the vicinity of the bottom surface of the cell culture vessel 7 with a gap therebetween.

The culture-medium supply means 6, which supplies the culture medium to such cell culture vessels 7, is connected to the supply ports of the cell culture vessels 7, thereby making it possible to configure a cell culture system. As the culture-medium supply means 6, for example, as shown in Fig. 21, it is possible to adopt a means in which a culture-medium storage vessel 152 and a supply port 151a are connected with a tubular member 153, such as a tube or the like, and a liquid feeding pump 154 is disposed in the tubular member 153.

Furthermore, the supply port may be disposed in the top surface of the cell culture vessel 7, and the culture medium may be dripped from the supply port onto the top surface of the culture medium by the culture-medium supply means 6. By doing so, backflow of the culture medium can be prevented, thereby making it possible to prevent the culture medium in the culture-medium holding vessel, which is disposed at an upstream side of the system, from being contaminated (the occurrence of contamination).

According to this cell culture system, the culture medium is supplied to the cell culture vessels 7 by the culture-medium supply means 6, and, when the culture medium in the cell culture vessels 7 exceeds a predetermined amount, the discharge siphon mechanism 6a discharges the culture medium to a predetermined height thereof on the basis of the siphon principle, thereby making it possible to intermittently replace the old culture medium with a new culture medium.

In the above-described embodiments and the modifications thereof, a form in which the culture medium is intermittently supplied and discharged according to the siphon principle has been illustrated; however, in a cell culture system using a porous membrane, such as a dialysis membrane, a solution, such as a dialysate solution, may be intermittently supplied and discharged according to the siphon principle.

Fig. 22A shows an example cell culture vessel used in this form. A culture medium and cells are contained in a cell-culture bag 162 made of a porous membrane. The cell-culture bag 162 is stored in a dialysis vessel 161 with being soaked in a solution, such as a dialysate solution. The dialysis vessel 161 is provided with a supply port 161a for supplying the solution to the inside and a discharge port 161b for discharging the solution to the outside. The dialysis vessel 161 and the cell-culture bag 162 function as the cell culture vessel in the above-described embodiments and modifications thereof.

Furthermore, Fig. 22B shows another example cell culture vessel. A culture medium and cells are contained in a culture vessel 164. A solution, such as a dialysate solution, is contained in a dialysis bag 163 made of a porous membrane, and the dialysis bag 163 is stored in the culture vessel 164 with being soaked in a culture medium. The dialysis bag 163 is provided with a supply port 163a for supplying the solution to the inside and a discharge port 163b for discharging the solution to the outside. The dialysis bag 163 and the culture vessel 164 function as the cell culture vessel in the above-described embodiments and modifications thereof.

This form can be applied to floating cells as well as adherent cells.

Specifically, according to the present invention, it is possible to provide a cell culture system including:
a solution storage means that holds a solution;
a temporary holding means that is coupled to the solution storage means and that is provided with a holding space for temporarily holding the solution supplied from the solution storage means and a discharge port for discharging the solution;
a solution supply means that is coupled to the discharge port of the temporary holding means and that supplies the solution discharged from the temporary holding means, to a cell culture vessel; and
a discharge means that discharges the solution from the cell culture vessel;
wherein the cell culture vessel is coupled to the solution supply means at a position lower in the direction of gravitational force than the solution supply means; and
the solution supply means is provided with a flow passage that allows the solution to be intermittently supplied from the temporary holding means to the cell culture vessel according to the siphon principle.

Furthermore, according to the present invention, it is possible to provide a cell culture system including:
a temporary holding means that is provided with a solution storage means that holds a solution, a holding space that is coupled to the solution storage means and that temporarily holds the solution supplied from the solution storage means, and a discharge port for discharging the solution;
a solution supply means that is coupled to the discharge port of the temporary holding means and that supplies the solution discharged from the temporary holding means, to a cell culture vessel; and
a discharge means that discharges the solution from the cell culture vessel,
wherein the cell culture vessel is coupled to the solution supply means at a position lower in the direction of gravitational force than the solution supply means; and
the solution supply means is provided with a supply gate that opens a flow passage when the culture medium in the holding space reaches a predetermined amount.

### {Reference Signs List}

1 incubator
2 temperature control means
3 culture-medium storage means
4 temporary holding means
4c backup discharge port
5 supply-rate adjusting means
5a liquid feeding pump
5b supply-rate adjusting means
6 culture-medium supply means
6a siphon mechanism
6c branching means
6d supply gate
7 cell culture vessel
8 culture-medium discharge means
8a waste-liquid holding means
19 control unit
20 tubular member (tube)
21 plate-like member
22 spherical (columnar) member
23, 25, 26 through-hole
24 shutter member
31 valve
32 ring member
33 ball
34 elastic member
41 valve
42 float
43 ring member
44 ball
45 string-like member
51 pressure applying means
61 saclike member
62, 64 isolation member
65 weight
71 negative-pressure supply means
81 pump
82 waste-liquid container
83 suction port
91 culture vessel
92 lid portion
95 supply tube portion
96 discharge tube portion
97 neck portion
100, 200, 300, 400, 500, 600, 700, 800, 900, 1000, 1100, 1200 cell culture system
101 convex portion
102 ring-like member
103 concave portion
111 disk-like member
112 lid body
113 ring-like member

## Claims

1. A cell culture system comprising:
a culture-medium storage means that holds a culture medium for culturing cells;
a temporary holding means that is coupled to the culture-medium storage means and that is provided with a holding space for temporarily holding the culture medium supplied from the culture-medium storage means and a discharge port for discharging the culture medium;
a culture-medium supply means that is coupled to the discharge port of the temporary holding means and that supplies the culture medium discharged from the temporary holding means, to a cell culture vessel; and
a discharge means that discharges the culture medium from the cell culture vessel,
wherein the cell culture vessel is coupled to the culture-medium supply means at a position lower in the direction of gravitational force than the culture-medium supply means; and
the culture-medium supply means is provided with a flow passage that allows the culture medium to be intermittently supplied from the temporary holding means to the cell culture vessel according to the siphon principle.

2. A cell culture system comprising:
a culture-medium storage means that holds a culture medium for culturing cells;
a temporary holding means that is coupled to the culture-medium storage means and that is provided with a holding space for temporarily holding the culture medium supplied from the culture-medium storage means and a discharge port for discharging the culture medium;
a culture-medium supply means that is coupled to the discharge port of the temporary holding means and that supplies the culture medium discharged from the temporary holding means, to a cell culture vessel; and
a discharge means that discharges the culture medium from the cell culture vessel,
wherein the cell culture vessel is coupled to the culture-medium supply means at a position lower in the direction of gravitational force than the culture-medium supply means; and
the culture-medium supply means is provided with a supply gate that opens a flow passage when the culture medium in the holding space reaches a predetermined amount.

3. A cell culture system according to claim 1, wherein the culture-medium supply means is provided with the flow passage that causes the culture medium before being supplied to the cell culture vessel to pass through a position that is higher in the direction of gravitational force than the discharge port of the temporary holding means and that is lower in the direction of gravitational force than the maximum height of the holding space.

4. A cell culture system according to claim 3,
wherein the discharge port is disposed in a side surface of the temporary holding means at the position of a predetermined height from a bottom surface of the temporary holding means; and
the culture-medium supply means forms the flow passage from the discharge port to the outside of the temporary holding means.

5. A cell culture system according to claim 3,
wherein the culture-medium supply means forms the flow passage from the discharge port of the temporary holding means to the inside of the temporary holding means; and
an end of the culture-medium supply means that is opposite from an end thereof that is connected to the discharge port of the temporary holding means has an opening in the vicinity of the bottom surface of the temporary holding means with a gap therebetween.

6. A cell culture system according to claim 2, wherein the supply gate opens the flow passage when the culture medium in the holding space reaches a predetermined weight.

7. A cell culture system according to claim 2, wherein the supply gate opens the flow passage when the top surface of the culture medium in the holding space reaches a predetermined height.

8. A cell culture system according to one of claims 1 to 7, wherein the cell culture vessel is provided with a discharge port for discharging the culture medium to the outside when the height of the top surface of the culture medium therein reaches a predetermined height.

9. A cell culture system according to one of claims 1 to 8, further comprising a supply-rate adjusting means that controls the supply rate of the culture medium supplied from the culture-medium storage means to the temporary holding means.

10. A cell culture vessel comprising: a supply port for supplying a culture medium; a discharge port for discharging the culture medium; and a tubular member that is connected to the discharge port,
wherein the tubular member forms a flow passage that allows the culture medium in the cell culture vessel to be intermittently discharged according to the siphon principle.

11. A cell culture vessel according to claim 10, wherein the tubular member forms a flow passage that passes through a position that is higher in the direction of gravitational force than the discharge port and that is lower in the direction of gravitational force than the maximum height of a space in the cell culture vessel.

12. A cell culture vessel according to claim 11,
wherein the discharge port is disposed in a side surface of the cell culture vessel at the position of a predetermined height from the bottom surface of the cell culture vessel; and
the tubular member forms a flow passage from the discharge port to the outside of the cell culture vessel.

13. A cell culture vessel according to claim 11,
wherein the tubular member forms a flow passage from the discharge port to the inside of the cell culture vessel; and
an end of the tubular member that is opposite from an end thereof that is connected to the discharge port has an opening in the vicinity of the bottom surface of the cell culture vessel with a gap therebetween.

14. A cell culture system comprising:
a cell culture vessel according to one of claims 10 to 13; and
a culture-medium supply means that supplies the culture medium from the supply port of the cell culture vessel to the inside thereof.
